Europäisches Patentamt

European Patent Office

Office européen des brevets

(11) Publication number: **0 159 418**
**B1**

(12) **EUROPEAN PATENT SPECIFICATION**

(45) Date of publication of patent specification: **09.05.90**

(51) Int. Cl.⁵: **C 12 N 15/00**

(21) Application number: **84200792.4**

(22) Date of filing: **04.06.84**

(30) Priority: **03.04.84 NL 8401048**

(43) Date of publication of application:
**30.10.85 Bulletin 85/44**

(45) Publication of the grant of the patent:
**09.05.90 Bulletin 90/19**

(84) Designated Contracting States:
**AT BE CH DE FR GB IT LI LU NL SE**

(54) **A process for the incorporation of foreign DNA into the genome of monocotyledonous plants.**

(56) References cited:

MOLECULAR BIOLOGY OF PLANT TUMORS,
1982, pages 537-543, Academic Press Inc.; J.
LEEMANS et al.: "Ti Plasmids and directed
genetic engineering"
IDEM
NATURE, vol. 303, 12th May 1983, pages
179-180, Macmillan Journals Ltd., London, GB;
A. HOEKEMA et al.: "A binary plant vector
strategy based on separation of vir- and T-
region of the Agrobacterium tumefaciens Ti-
plasmid"
JOURNAL OF BACTERIOLOGY, vol. 154, no. 2,
May 1983, pages 693-701, American Society for
Microbiology; J. HILLE et al.: "Site-directed
mutagenesis in Escherichia coli of a stable R772:
Ti cointegrate plasmid from Agrobacterium
tumefaciens"
NATURE, vol. 307, 12th January 1984, pages
108-109, Macmillan Journals Ltd., London, GB;
R. FLAVELL et al.: "Prospects for transforming
monocot crop plants"
NATURE, vol. 311, 25th October 1984, pages
763-764; G.M.S. HOOYKAAS-VAN SLOGTEREN
et al.: "Expression of Ti plasmid genes in
monocotyledonous plants infected with
Agrobacterium tumefaciens"

(73) Proprietor: **RIJKSUNIVERSITEIT LEIDEN**
Stationsweg 46 P.O. Box 9500
NL-2300 RA Leiden (NL)

(73) Proprietor: **Schilperoort, Robbert Adriaan, Prof.
Dr.**
Anthonie Duycklaan 10c
NL-2334 CD Leiden (NL)

(72) Inventor: **Hooykaas, Paul Jan Jacob, Dr.**
Condorhorst 126
NL-2317 AW Leiden (NL)
Inventor: **van Slogteren, Geertrui Martha Sibrig,
Dr.**
Condorhorst 126
NL-2317 AW Leiden (NL)
Inventor: **Schilperoort, Robbert Adriaan, Prof.
Dr.**
Anthonie Ducklaan 10c
NL-2334 CD Leiden (NL)

(74) Representative: **Kooy, Leendert Willem et al**
OCTROOIBUREAU VRIESENDORP & GAADE
P.O. Box 266
NL-2501 AW The Hague (NL)

Courier Press, Leamington Spa, England.

## EP 0 159 418 B1

(56) References cited:

The Botanical Review, Vol.42, no.4, 1976, pp.
389-403, 410-413, 462-466
Nature, Vol. 283, 1980, pp. 794-796
Phytopath.Z, Vol. 113, 1985, pp. 81-85
Plant Molecular Biology, Vol,7, 1986, pp. 43-50
Nature, Vol. 325, 1987, 177-179
Nature, Vol.327, 1987, pp. 529-532
The EMBO Journal, Vol.3, 1984, pp. 3043-3047
J.Gen.Microbiol., Vol115, pp.479-489
Ann.Rev.Microbiol. Vol.35, 1981, pp. 531-565
The Botanical Review, Vol. 47, no. 2, 1981, pp.
147-194
Int. J. Syst. Bacteriol., Vol.27, 1977, pp. 222-240
J. Bacteriol, Vol. 169, no. 4, 1987, pp. 1745-1746
Plant Molecular Biology, Vol. 11, 1988, pp. 35-43
The EMBO Journal, Vol. 3, no. 13, 1984, pp.
3039-3041
Plant Molecular Biology, Vol. 12, 1989, pp. 31-40
THE PROKARYOTES ; HANDBOOK ON
HABITATS, ISOLATION AND IDENTIFICATION
OF BACTERIA, 1981 (EDS. M.P. STARR ET AL.),
SPRINGER VERLAG, CHAPTER 68, THE GENUS
AGROBACTERIEUM, PP. 842-852
Proc.Natl.Acad.Sci.USA, vol.84,1987,pp. 5345-
5349
BIOTECHNOLOGY, VOL. 6, 1988, PP.185-189

## Description

The invention relates to a process for the incorporation of foreign DNA into the genome of monotyledonous plants, by infecting the plants or incubating the plant protoplasts with bacteria suitable or made suitable for that purpose.

A number of bacteria, such as *Agrobacterium tumefaciens* and *Agrobacterium rhizogenes* are by nature capable of manipulating plant cells genetically. The said bacteria adhere to the plant cell wall and subsequently introduce a fragment of DNA into the plant cell via a process still unexplained. This DNA fragment (the T-DNA) is as T-region in the bacteria a part of a large plasmid (120—150 Mdal.), which in case of *A. tumefaciens* is called Ti-plasmid and in case of *A. rhizogenes* Ri-plasmid. The T-DNA is then integrated into the nucleus genome of the plant cell (Thomashow, M. F., et al. (1980), Proc. Nat.Acad.Sci. USA 77: 6448—6452). Genes positioned in the T-DNA express·therein and see to it that the plant cell will behave like a tumour cell (Ooms, et al (1981), Gene 14: 33—50). Meanwhile methods have been developed to couple no matter which DNA to the T-region in such a way that this can be subsequently be introduced into the plant cell simultaneously with the T-region (Hille, J. et al (1983), J. Bacteriol. 154: 693—701 and Hoekema, A. et al (1983), Nature 303: 179—180). From the T-region the *onc*-genes can be removed (Hille J., et al (1983), Plant Mol. Biol. 2: 155—163 and Zambryski, P., et al (1983), EMBO J. 2: 2143—2150). This results in vectors which are indeed transmitted by the bacteria to the plant cell, but which are no longer oncogenic (Hille, J., et al (1983), Plant Mol. Boil. 2: 155—163 and Zambryski, P., et al (1983), EMBO J. 2: 2143—2150). Plant cells transformed with these "disarmed" vectors can regenerate to plants having new properties (encoded by the genes introduced into the T-region (Zambryski P., et al (1983), EMBO J. 2: 2143—2150). Stable cointegrate plasmids constructed from plasmid and plasmid pTiB6 are known from J. Bacter. May 1983 Vol. 154, No. 2, pp. 693—701).

If *Rhizobium* bacteria are provided with plasmids such as meant above, or plasmids derived therefrom, they are also capable of the genetic manipulation of plant cells (Hooykass et al 1977). Now, it has appeared from experiments with mutants of *Agrobacterium* and with strains of *Rhizobium* occurring in nature that in addition to the said plasmids also particular other sequences of the bacterial DNA are necessary, for instance the sequence responsible for the adherence of the bacteria to the cell wall. The above means that *Agrobacterium* is not the only organism capable of inserting of T-DNA into the plant genome.

There are other bacteria which are capable hereof if they are provided with the plasmids or derivatives thereof ultimately originating from *Agrobacterium*. A *conditio sine qua non* is in this respect that they avail of the necessary attendant DNA-sequences such as described above, or functional analogues thereof and that no inherent impediments for the transfer of DNA are present.

It also appeared in research work of our group performed earlier that it is not absolutely necessary that the plasmid genes for virulence and the T-DNA are actually positioned on plasmids. For instance the T-DNA may be situated on the bacterial chromosome, whilst the plasmid genes for virulence are positioned on an independent plasmid.

According to literature data the said systems can be used as a vector only for dicotyledonous plants, since only dicotyledonous plants respond to Agrobacteria with tumour formation (Lippincott, J. A. & Lippincott, B, B. (1975), Ann. Rev. Microbiol. 29:377—407).

The fact that the cell wall of monocotyledonous plant differs from that of dicotyledonous plants would be the cause (Lippincott, J. A. & Lippincott, B. B. (1980).

In "Bacterial Adherence in Receptors and Recognition" (E. H. Beachley, ed.), pages 377—396 and Rao, S. S. et al (1982), Physiol. Plant. 56: 374—380). So, the prevailing opinion is that the said plasmids are not suitable as a vector for the genetic manipulation of monocotyledonous plants (Flavell, R. & Mathias, R. (1984), Nature 307: 108—109). But among the monocotyledonous plants there are highly important agricultural and horticultural crops, such as the grains and bulbous plants like tulip, daffodil and lily. The research performed described hereinunder was carried out in order to see whether the prevailing opinion about the absence of possibilities to use the Ti-plasmid as a vector for monocotyledonous plants was correct indeed. In this research various species of monocotyledonous plants were infected with *A. tumefaciens* strains, which either possessed various type of Ti- or Ri-plasmids separately or accomodated a combination of such plasmids. Surprisingly, it appeared from the experiments that cells of monocotyledonous plants were positively transformed by *A. tumefaciens*. This shows for the first time that the Ti-plasmid (or plasmids derived therefrom) is suitable for the genetic manipulation of monocotyledonous plants.

### Experiment

In the T-DNA in addition to *onc*-genes, which see to phytohormone synthesis (Ooms G., et al (1981), Gene 14: 33—50 and Garfinkel D. J., et al (1981), Cell 27: 143—153, there are also genes which encode for enzymes which see to the biosynthesis of "opines". These are tumour specific amino acid derivatives, such as octopine and nopaline Tempé, J. & Goldman, A. (1982) in: "Molecular Biology of Plant Tumours" (G. Kahl & J. Schell, eds.), pages 427—449. Opines do not occur in normal plant cells and so can be considered as specific indicators for incorporation and expression of T-DNA in the plant cell, even if there would not be question of transformation leading to the formation of tumour cells. There are various types of T-DNA which carry genes for enzymes which see to the biosynthesis of different opines. The most well-known are the octopine and resp. nopaline T-DNA's.

A great number of tests was now carried out in which monocotyledonous plants from the family of *Liliaceae* (Chlorophytum capense) and the family of Amaryllidaceae (*Narcissus* cv. Paperwhite) were wounded and subsequently infected with various *A.* tumefaciens strains. The strains used were; LBA288 (avirulent; no Ti-plasmid), LBA1010 (contains an octopine Ti-plasmid), LBA2318 (contains a nopaline Ti-plasmid), LBA1020 (contains a Ri-plasmid), LBA2347 (contains a nopaline Ti-plasmid+Ri-plasmid), LBA1516 (contains an octopine Ti-plasmid with a Vir-mutation; avirulent). After a few weeks plant material was removed from the infection sites and analysed for opines (Otten, L. A. B. M. & Schilperoort, R. A. (1978) Biochim. Biophys. Acta 527: 497—500). It is to be remarked that after infection with virulent strains often a slight thickening around the wound was to be observed. Thick tumours were not formed. In the case of infection with avirulent strains no thickening was observed at all.

Plant material isolated from non-wounded or wounded, but non-infected monocotyledonous plants appeared to contain neither octopine nor nopaline. Opines were not discovered either in plant material isolated from plants infected with the avurulent strains of LBA288 or LBA1516. However, plant cells obtained from wound sites infected with virulent *A. tumefaciens* strains appeared to contain nearly always opines. After infection with a strain with an octopine Ti-plasmid (LBA1010) octopine appeared to be present, whilst after infection with strains with a nopaline Ti-plasmid (LBA2318, LBA2347) nopaline appeared to be present.

The results obtained with strain LBA2347 gave the most reproducible positive results. All these data together tender evidence that *A. tumefaciens* is capable of transmitting T-DNA to monocotyledonous plant cells, and furthermore that T-DNA genes (at any rate those involved for the enzymes in the biosynthesis of opines) express in monocotyledonous plant cells. Consequently, it can be concluded that the Ti-plasmid is suitable as a vector for monocotyledonous plant cells. Furthermore, the conclusion can be drawn that the regulator zones which see to the expression of the opine synthesis are funcational regions and so suitable as building-stones for making chimaeric genes which should express in monocotyledonous plant-cells.

Consequently, the invention relates to a process for incorporating foreign DNA into monocotyledonous plants by infecting the plants or incubating plant protoplasts with bacteria suitable or made suitable for that purpose which avail of one or more tumour-inducing plasmids, for instance Ti- or Ri-plasmids, or derivatives thereof, or parts thereof, originally originating from Agrobacterium bacteria.

Preferably, use is made of the *Agrobacterium* bacteria, which has at least one plasmid which has the Vir-region of a Ti (tumour inducing) plasmid but no T-region and at least one other plasmid which·has a Ti-region with built in it foregin DNA but no Vir-region.

For the Agrobacterium bacteria, which are suitable for the processing according to the invention as well as for the way in which these bacteria can be obtained rights were already claimed in the non-prepublished European patent application 84200239.6 the teachings of which is included herein in their entirety by reference.

For expressing foreign genes in monocotyledonous plant cells use can be made of the regulator regions of T-DNA-genes, in particular the genes for octopine synthesis and nopaline synthesis.

## Claims

1. A process for the incorporation of foreign DNA into the genome of monocotyledonous plants by infecting the monocotyledonous plants for incubating the protoplasts thereof with Agrobacterium or Rhizobium bacteria containing a virulence region and at least one T-region originating from a Ti-plasmid or a Ri-plasmid or both, which T-region is provided with said foreign DNA.

2. A process according to claim 1, characterized in that the bacteria used are provided with a stable cointegrate plasmid constructed from a plasmid R772 and a plasmid pTiB6 with foreign DNA incorporated into the T-region of the latter.

3. A process according to any of the preceding claims, characterized in that bacteria are used, which contain at least one plasmid, which has the Vir-region of a Ti(tumor-inducing) plasmid but no T-region, and at least one other plasmid, which has a T-region with incorporated foreign DNA but no Vir-region.

4. A process according to any of the preceding claims, characterized in that the regulator regions positioned before and after the protein encoding regions of T-DNA-genes in particular the genes for octopine synthesis and nopaline synthesis are used for expressing foreign DNA in monocotyledonous plant cells.

## Patentansprüche

1. Verfahren zum Einbau von fremder DNS in das Genom von monocotyledonen Pflanzen durch Infizieren der monocotyledonen Pflanzen oder Ikubieren der Protoplasten dieser Pflanzen mit Agrobacterium- oder Rhizobium-Bakterien, enthaltend eine Virulenzregion und mindestens eine T-Region, die von einem Ti-Plasmid oder einem Ri-Plasmid oder beiden stammt, wobei die T-Region mit der genannten fremden DNS zur Verfügung gestellt wird.

2. Verfahren nach Anspruch 1, dadurch gekennzeichnet, daß die angewandten Bakterien ein stabiles, mitintegriertes Plasmid aufweisen, das aus einem Plasmid R772 und einem Plasmid pTiB6 besteht, wobei fremde DNS in die T-Region der zuletzt genannten eingebaut ist.

3. Verfahren nach einem der vorangehenden Ansprüche, dadurch gekennzeichnet, daß Bakterien angewandt werden, die mindestens ein Plasmid enthalten, das die Vir-Region eines Ti(Tumorinduzierenden)-Plasmids, aber keine T-Region aufweist und mindestens ein anderes Plasmid, das eine T-Region besitzt mit eingebauter fremder DNS, aber keine Vir-Region.

4. Verfahren nach einem der vorangehenden Ansprüche, dadurch gekennzeichnet, daß die vor und hinter den Protein-codierenden Regionen der T-DNS-Gene, insbesondere der Gene für die Octopinsynthese und Nopalinsynthese, angeordneten Regulatorregionen verwandt werden, um die fremde DNS in monocotyledonen Pflanzenzellen zum Ausdruck zu bringen.

**Revendications**

1. Procédé d'incorporation d'ADN étranger dans le génome des plantes monocotylédones en infectant les plantes monocotylédones ou en incubant leurs protoplastes avec des bactéries Agrobacterium ou Rhizobium contenant une région de virulence et au moins une région T provenant d'un plasmide Ti ou d'un plasmide Ri ou des deux, région T qui comporte ledit ADN étranger.

2. Procédé selon la revendication 1, caractérisé en ce que les bactéries utilisées comportent un plasmide co-intégré stable formé à partir d'un plasmide R772 et d'un plasmide pTiB6 avec ADN étranger incorporé dans la région T de ce dernier.

3. Procédé selon la revendication 1 ou 2, caractérisé en ce qu'on utilise des bactéries qui contiennent au moins un plasmide ayant une région Vir ou un plasmide Ti (inducteur de tumeurs) mais pas de région T, et au moins un autre plasmide qui comporte une région T dans laquelle est incorporé l'ADN étranger mais sans région Vir.

4. Procédé selon l'une quelconque des revendications précédentes, caractérisé en ce qu'on utilise des régions régulatrices disposées avant ou après les régions de codage des protéines des gènes d'ADN-T, en particulier les gènes pour la synthèse des octopines et la synthèse des nopalines, afin d'exprimer l'ADN étranger dans les cellules des plantes monocotylédones.